# EUROPEAN PATENT APPLICATION

(11) **EP 2 530 186 A1**
(43) Date of publication of application: **05.12.2012**
(21) Application number: 11737082.5
(22) Date of filing: 27.01.2011
(51) Int. Cl.: C25B 3/08, C07C 303/22, C07C 309/06

(54) **PROCESS FOR PRODUCING PERFLUOROBUTANESULFONIC ACID SALT**

(30) Priority: 27.01.2010 JP 2010015641
(71) Applicant: Mitsubishi Materials Corporation, Chiyoda-ku Tokyo 100-8117 (JP); Mitsubishi Materials Electronic Chemicals Co., Ltd., Akita-shi Akita 010-8585 (JP)
(72) Inventor: UOTANI Masakazu, Akita-shi Akita 010-8585 (JP); KAMIYA Takeshi, Akita-shi Akita 010-8585 (JP); HONDA Tsunetoshi, Akita-shi Akita 010-8585 (JP)
(74) Representative: Gille Hrabal
(86) International application number: PCT/JP2011/051581
(87) International publication number: WO 2011/093371

(57) **Abstract**

A process for producing a perfluorobutanesulfonic acid salt (PFBS salt) is provided. By the process, the yield is improved and PFOS salt content is reduced. Electrochemical fluorination is conducted in a reaction solution comprising anhydrous hydrogen fluoride to generate perfluorobutanesulfonyl fluoride, and the fluoride its hydrolyzed to produce a perfluorobutanesulfonic acid salt. The process for producing a perfluorobutanesulfonic acid salt includes: a step in which liquid phase components generated in the electrochemical fluorination cell are withdrawn and a first a perfluorobutanesulfonic acid salt fraction is prepared therefrom; and a step in which gaseous phase components discharged from the electrochemical fluorination cell are collected and a second perfluorobutanesulfonic acid salt fraction is prepared therefrom. The reaction solution in the electrochemical fluorination cell is regulated so as to have a perfluorooctanesulfonyl fluoride content of 500 ppm or lower by withdrawing the liquid phase components from the electrochemical fluorination cell.

## Description

### TECHNICAL FIELD

The present invention relates to a method of producing perfluorobutanesulfonic acid salt.
Priority is claimed on Japanese Patent Application No. 2010-015641, filed January 27, 2010, the content of which is is incorporated herein by reference.

### BACKGROUND ART

Conventionally, the perfluorobutanesulfonic acid salt represented by a formula, C₄F₉SO₃· M (M is a cation, such as Li, Na, K, NH₄, or the like) is suitably used in the production of a photo-acid-generator or a flame retardant for polycarbonate resin as a raw material.

As a process for producing perfluoroalkanesulfonate salt represented by a formula, CₙF₂ₙ₊₁SO₃· M (n is an integer of 1 or more, and M is a cation components), a method, in which alkanesulfonyl fluoride represented by a formula CₙH₂ₙ₊₁SO₂F (n is an integer of 1 or more) is electrochemical fluorination in anhydrous hydrogen fluoride to produce perfluoroalkanesulfonyl fluoride, and the perfluoroalkanesulfonyl fluoride is hydrolyzed to produce perfluoroalkanesulfonate salt, is known (Patent Literature 1).

However, a composition of alkanesulfonyl fluoride represented by the formula CₙH₂ₙ₊₁SO₂F and having a less number of carbon atoms (for example, the n is 1 to 3 in the formula) is exhausted from the electrochemical fluorination cell in the production method described in Patent Literature 1, since the boiling point of the products is low. For that reason, a method, in which a mixture of an alkaline solution and a surfactant is used as an absorbing solution in order to recover the produced gas containing the perfluoroalkanesulfonyl fluoride by absorbing it efficiently with the absorbing solution, is disclosed in Patent Literature 2.

On the other hand, a composition of perfluoroalkanesulfonyl fluoride represented by the formula CₙH₂ₙ₊₁SO₂F and having a larger number of carbon atoms (for example, the n is 4 or more in the formula) is generated as an liquid phase component. For that reason, a method, in which the perfluoroalkanesulfonyl fluoride is extracted as an liquid phase component, is disclosed in Patent Literature 3.

As a perfluorobutanesulfonic acid salt (PFBS salt) production method capable of increasing its yield is disclosed in Patent Literature 4. In the perfluorobutanesulfonic acid salt (PFBS salt) production method disclosed in the Patent Literature 4, butyl sulfonyl fluoride, tetrahydrothiophene- 1, 1 -dioxyde (sulfolane), 2,5-dihydrohiophene-1,1 -dioxyde (sulfolene), or a mixture of the above-mentioned components is used as a starting material. Then, perfluorobutanesulfonyl fluoride (hereinafter referred as "PBF") is produced by electrochemical fluorination of butyl sulfonyl fluoride, tetrahydrothiophene-1,1-dioxyde (sulfolane), 2,5-dihydrohiophene-1,1-dioxyde (sulfolene), or a mixture of them in the presence of perfluorooetanesulfonyl fluoride (C₈F₁₇SO₂F; hereinafter referred as "POF"). Then, PBF is extracted from a electrochemical fluorination cell as a liquid phase component. It is believed that the yield of perfluorobutanesulfonic acid salt can be improved by using the method.

### [Related Art Document]

Patent Document 1: US Patent (Granted) Publication No. 2732398
Patent Document 2: Japanese Unexamined Patent Application, First Publication No. 2009-179836
Patent Document 3: Japanese Unexamined Patent Application, First Publication No. 2002-38288
Patent Document 4: Japanese Unexamined Patent Application, First Publication No. H6-49674

### DISCLOSURE OF INVENTION

### [Problems to be Solved by the Invention]

In the production method described in Patent Literature 4, there is an improvement of the yield of perfluorobutanesulfonic acid salt due to the presence of POF. However, it is still problematic since the improvement is not sufficient.

In addition, in the production method described in the Patent Literature 4, purification of PBF is difficult, since the produced PBF is obtained as a mixture containing POF. As a result, perfluorooctanesulfonic acid salt (PFOS salt) is mixed in the produced perfluorobutanesulfonic acid salt as an impurity. The PFOS salt is a chemical that is highly stable and resistant to decomposition in an environment. Therefore, an environmental accumulation is concerned. Accordingly, it has been demanded to reduce the PFOS salt content in the perfluorobutanesulfonic acid salt in a response to an international regulation as a new environment pollutant in recent years.

The present invention is made under the above-mentioned circumstance. The purpose of the present invention is to provide a producing process capable of increasing the yield of the perfluorobutanesulfonic acid salt and reducing the perfluorooctanesulfonic acid salt (PFOS salt) content in the perfluorobutanesulfonic acid salt.

### [Means for Solving the Problems]

In order to achieve the purpose, the present inventors conducted extensive studies and found the following fact, completing the present invention. A part of produced PBF is exhausted from the system as an exhaust gas with the co-produced hydrogen gas, since the boiling point of the PBF produced by the electrochemical-fluorination is about 65°C. Also, the amount of POF mixed in the gaseous phase can be reduced by decreasing the POF content in the reaction solution lower than a predetermined value.

The first aspect of the present invention is a process for producing perfluorobutanesulfonic acid salt including the steps of: electrochemical fluorination, in which a liquid phase component and a gaseous phase component containing perfluorobutanesulfonyl fluoride are generated by electrochemical fluorination an electrolytic material consisting of one or more selected from the group consisting of butane sulfonyl fluoride, tetrahydrohiophene-1,1 -dioxide (sulfolane), and 2,5-dihydrohiophene- 1, 1- -dioxide (sulfolene) in a reaction solution made of anhydrous hydrogen fluoride in a electrochemical fluorination cell; preparing a first perfluorobutanesulfonate fraction, in which the first perfluorobutanesulfonate fraction is prepared by withdrawing the liquid phase component generated in the electrochemical fluorination cell in the step of electrochemical fluorination and hydrolyzing the perfluorobutanesulfonyl fluoride contained in the liquid phase component; and preparing a second perfluorobutanesulfonate fraction, in which the second perfluorobutanesulfonate fraction is prepared by collecting the gaseous phase component that is exhausted from the electrochemical fluorination cell and generated in the step of electrochemical fluorination and hydrolyzing the perfluorobutanesulfonyl fluoride contained in the gaseous phase component, wherein a perfluorobutanesulfonyl fluoride content in the reaction solution is controlled to be 500 ppm or less by withdrawing the liquid phase component from the electrochemical fluorination cell in the step of electrochemical fluorination.
In the process for producing perfluorobutanesulfonic acid salt of the first aspect of the present invention, the step of preparing the first perfluorobutanesulfonate fraction may include a process of purifying perfluorobutanesulfonyl fluoride, in which perfluorobutanesulfonyl fluoride is purified by distilling the liquid phase component before hydrolyzing the perfluorobutanesulfonyl fluoride contained in the liquid phase component; and the purified perfluorobutanesulfonyl fluoride may be may be hydrolyzed by an alkaline solution.
Also, in the process for producing perfluorobutanesulfonic acid salt of the first aspect of the present invention, the step of preparing the second perfluorobutanesulfonate fraction may include a process of removing hydrogen fluoride gas, in which hydrogen fluoride gas is removed from the gaseous phase component before hydrolyzing the perfluorobutanesulfonyl fluoride contained in the gaseous phase component; the perfluorobutanesulfonyl fluoride in the gaseous phase component, the hydrogen fluoride gas of which being removed, may be hydrolyzed by gas-liquid contacting to the alkaline solution; and the second perfluorobutanesulfonate fraction may be collected by the alkaline solution.
Also, in the process for producing perfluorobutanesulfonic acid salt of the first aspect of the present invention, a perfluorooctanesulfonic acid salt (PFOS salt) content in the first perfluorobutanesulfonate fraction and a perfluorooctanesulfonyl fluoride salt (PFOS salt) content in the second perfluorobutanesulfonate fraction may be 50 ppm or less.
Also, a perfluorooctanesulfonyl fluoride salt (PFOS salt) content in the first perfluorobutanesulfonate fraction and a perfluorooctanesulfonyl fluoride salt (PFOS salt) content in the second perfluorobutanesulfonate fraction may be 10 ppm or less.
The second aspect of the present invention is a perfluorobutanesulfonic acid salt-containing composition produced by the process for producing perfluorobutanesulfonic salt of the first aspect of the present invention, wherein: a perfluorobutanesulfonic acid salt concentration in the perfluorobutanesulfonic acid salt-containing composition is 99% by mass or more; and a perfluorooctanesulfonic acid salt (PFOS salt) content is 50 ppm or less.
In the perfluorobutanesulfonic acid salt-containing composition of the second aspect of the present invention, a perfluorooctanesulfonic acid salt (PFOS salt) content may be 10 ppm or less.
Also, the perfluorobutanesulfonic acid salt-containing composition of the second aspect of the present invention may contain 200 ppm or less of a perfluoroalkanesulfonate salt represented by a formula CₙF₂ₙ₊₁SO₃· M, wherein n indicates an integer from 1 to 3, and M indicates a cation component.

### [Effects of the Invention]

The process for producing perfluorobutanesulfonic acid salt of the first aspect of the present invention includes the steps of preparing the first perfluorobutanesulfonic acid salt fraction and the second perfluorobutanesulfonic acid salt fraction. In the step of preparing the first perfluorobutanesulfonic acid salt fraction, a liquid phase component produced in can electrochemical fluorination cell is withdrawn, and the first perfluorobutanesulfonic acid salt fraction is prepared. In the step of preparing the second perfluorobutanesulfonic acid salt fraction, a gaseous phase component exhausted from the electrochemical fluorination cell is collected, and the second perfluorobutanesulfonic acid salt is prepared. Because of the configuration described above, perfluorobutanesulfonyl fluoride, which had been exhausted conventionally as the gaseous phase component from the electrochemical fluorination cell to the outside of the system with hydrogen, can be collected to improve the yield of perfluorobutanesulfonic acid salt. In addition, the POF content mixed in the gaseous phase can be reduced by withdrawing a liquid phase from the electrochemical fluorination cell in such a way that perfluorooetanesulfonyl fluoride (POF) in the reaction solution in the electrochemical fluorination cell is kept 500 ppm or less. Because of the configuration described above, perfluorooctanesulfonic acid salt (PFOS salt) content in the second perfluorobutanesulfonic acid salt fraction can be suppressed at 10 ppm or less.

The perfluorobutanesulfonic acid salt-containing composition of the second aspect of the present invention is produced by the process for producing perfluorobutanesulfonic acid salt of the first aspect of the present invention. Therefore, the concentration of perfluorobutanesulfonic acid salt contained in the perfluorobutanesulfonic acid salt-containing composition can be kept at 99% or higher by mass ratio. Also, the perfluorooctanesulfonic acid salt (PFOS salt) content in the perfluorobutanesulfonic acid salt-containing composition can be kept at 50 ppm or less, or 10 ppm or less.

### BRIEF DESCRIPTION OF THE DRAWINGS

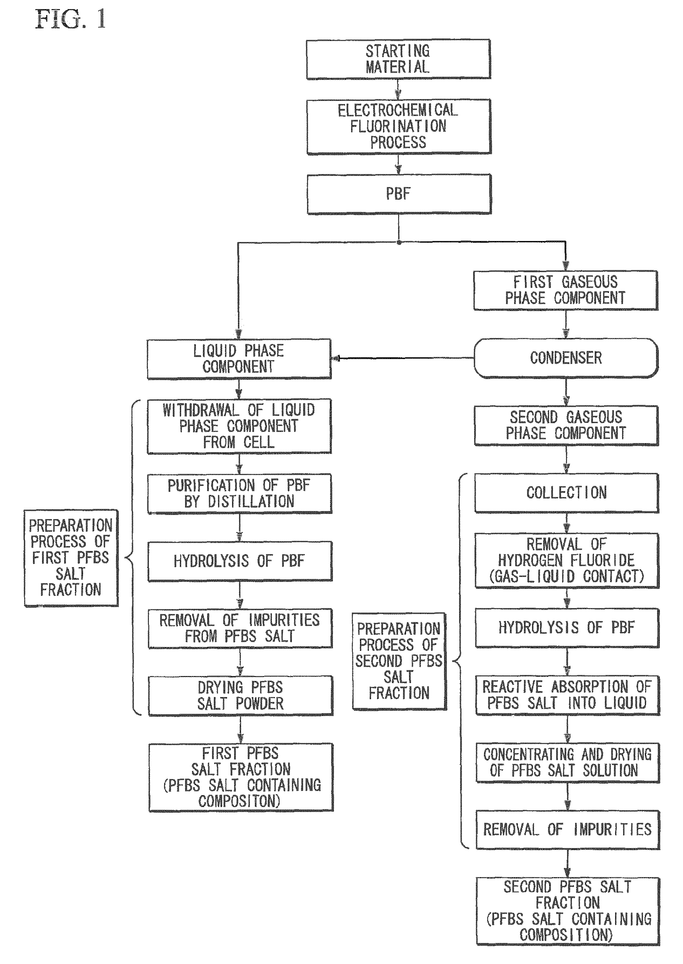

FIG. 1 is a flowchart showing the flow of components, steps, and processes in the process for producing perfluorobutanesulfonic acid salt (Examples 1 and 2).

### BEST MODE FOR CARRYING OUT THE INVENTION

The process for producing perfluorobutanesulfonic acid salt of the first embodiment of the present invention is explained below.
The process for producing perfluorobutanesulfonic acid salt of the first aspect of the present invention includes a step, in which perfluorobutanesulfonyl fluoride (PBF) is generated (step of electrochemical fluorination), a step, in which the first perfluorobutanesulfonic acid salt is prepared by withdrawing a liquid phase component produced in the electrochemical fluorination cell (step of preparing the first perfluorobutanesulfonic acid salt fraction), and a step, in which the second perfluorobutanesulfonic acid salt is prepared by collecting a gaseous phase component exhausted from the electrochemical fluorination cell (step of preparing the second perfluorobutanesulfonic acid salt fraction). In addition, perfluorooctanesulfonyl fluoride (POP) in a reaction solution in the electrochemical fluorination cell is controlled to be 500 ppm or less by withdrawing the liquid phase component from the electrochemical fluorination cell. Each step is explained in detail below in reference to FIG. 1.

### [Starting Material]

In the process for producing perfluorobutanesulfonic acid salt of the present embodiment, butyl sulfonyl fluoride (C₄H₉SO₂F), tetrahydrothiophene-1,1-dioxyde (sulfolane), 2,5-dihydrohiophene-1,1 -dioxyde (sulfolene), or a mixture of them can he used.
For example, butyl sulfonyl fluoride, which is one of the starting materials, can be produced easily by fluorine-substituting butanesulfonyl chloride with potassium fluoride or the like as shown in the formula (1) shown below.

C₄H₉SO₂Cl + KF → C₄H₉SO₂F + KCl (1)

### [Electrochemical Fluorination Process]

The electrochemical fluorination process is a step producing perfluorobutanesulfonyl fluoride (C₄F₉SO₂F; PBF) from the above-mentioned materials.
Specifically, the butyl sulfonyl fluoride is used as a material, it is placed in an electrochemical fluorination cell with anhydrous hydrogen fluoride, and electrochemical fluorination is performed under the ordinary pressure in a nitrogen gas atmosphere, for example. By following procedure described above, the alkyl group of the butyl sulfonyl fluoride is fluorine-substituted, and PBF is produced as in the formula (2) shown below.

C₄H₉SO₂F+ + 9HF → C₄F₉SO₂F + 9H₂↑ (2)

A reaction formula, in which perfluorobutanesulfonyl fluoride (C₄F₉SO₂F: PBF) is produced using tetrahydrothiophene-1,1-dioxyde (sulfolane) as a material, is shown as the formula (3) shown below.

C₄H₈SO₂ + 10HF → C₄F₉SO₂F + 9H₂↑ (3)

A part of the produced PBF is exhausted outside of the system as the first gaseous phase component with co-produced hydrogen, perfluoroalkanes and sulfonyl difluoride which are the decomposed products, nitrogen which is the substituting and anhydrous hydrogen fluoride which is the solvent of electrochemical fluorination from the cell in a gas form. Here, by allowing the first gaseous component to pass a condenser in which a coolant at -10°C to -40°C is circulating, a part of the PBF that is exhausted from the electrochemical fluorination cell as the gas is liquefied with the co-presenting hydrogen fluoride and returned to the electrochemical fluorination cell.
Then, the liquefied PBF that is returned to the electrochemical fluorination cell forms a liquid phase in the lower layer of the reaction solution in the electrochemical fluorination cell. However, a part of the produced PBF is not liquefied even after passing the condenser and exhausted to the outside of the system as the second gaseous phase component with the produced hydrogen gas. Based on an investigation performed by the present inventors, it has been confirmed that the PBF production ratio (weight ratio) in liquid phase to gaseous phase is roughly 3 to 2.

In the electrochemical fluorination process, perfluorooctanesulfonyl fluoride (C₈F₁₇SO₂F; POF) is produced in a side reaction.
In addition, perfluoroalkanesulfonyl fluoride represented by a formula CₙF₂ₙ₊₁SO₂F (n is an integer of 1 to 3) is produced due to breakage of the carbon chain of the material.
Furthermore, perfluoroalkanes represented by a formula CₙF₂ₙ₊₂ or sulfonyl difluoride (SO₂F₂) is produced due to decomposition the material, a reaction intermediate, and the produced PBF.

In the electrochemical fluorination process, hydrogen fluoride escapes from the electrochemical fluorination cell accompanied with hydrogen. Therefore, it is preferable to supply the material and anhydrous hydrogen fluoride continuously to the electrochemical fluorination cell.

### [Preparation process of the first perfluorobutanesulfonic acid salt fraction]

The preparation process of the first perfluorobutanesulfonic acid salt fraction is a process, in which the liquid phase component produced in the electrochemical fluorination cell is withdrawn and the first perfluorobutanesulfonic acid salt fraction is prepaced.
Specifically, it includes a process in which the liquid phase component is withdrawn, a process in which perfluorobutanesulfonyl fluoride (PBF) is purified by distilling the withdrawn liquid phase component, and a process in which perfluorobutanesulfonic acid salt is produced by hydrolyzing the purified PBF with an alkaline solution.

First, the liquid phase component produced in the electrochemical fluorination cell is withdrawn. The PBF cooled by the condenser is separated from the reaction solution in the electrochemical fluorination cell, and stays in the lower layer side of the reaction solution as a liquid phase. Thus, the liquid phase component is withdrawn from a withdrawal nozzle provided to the lower part of the electrochemical fluorination cell.

Next, PBF is purified by distilling the withdrawn liquid phase component. Specifically, the liquid phase component is heated at 60 to 90°C with a distillation tower, for example. Here, the boiling point of perfluorooctanesulfonyl fluoride (POF) dissolved in the PBF is about 160°C, whereas the boiling point of PBF is about 65°C. By separating POF ufilizing the difference of the boiling points, PBF can be purified, the POF content in the PBF being reduced to 50 ppm or less at least, or preferably to 10 ppm or less.

Next, perfluorobutanesulfonic acid salt is produced by hydrolyzing the purified PBF with an alkaline solution.
As the alkaline solution, lithium hydroxide (LiOH), sodium hydroxide (NaOH), and potassium hydroxide (KOH) can be used, for example.
The hydrolyzing reaction by the alkaline solution is shown as a formula (4) below. Examples of the perfluorobutanesulfonic acid salt produced in the reaction shown in the formula (4) are perfluorobutanesulfonate lithimn salt (LFBS salt), perfluorobutanesulfonate sodium salt, and perfluorobutanesulfonate potassium salt (KFBS salt).

As shown in the formula (4) below, PBF is converted to perfluorobutanesulfonic acid salt (C₄F₉SO₃M) by reacting with the hydroxide (MOH) in the alkaline solution.

C₄F₉SO₂F + 2MOH → C₄F₉SO₃M + MF +H₂O (4)

In the formula (4), M is any one of Li, Na, and K.

Next, impurities (MF) are removed from the reaction solution containing the produced perfluorobutanesulfonic acid salt, and obtained powders of the PFBS salt are dried.
By following the above-explained processes, the first perfluorobutanesulfonic acid salt fraction is prepared.

### [Preparation process of the second perfluorobutanesulfonic acid salt fraction]

The preparation process of the second perfluorobutanesulfonic acid salt fraction is a process in which the gaseous phase component exhausted from the electrochemical fluorination cell is collected and the second perfluorobutanesulfonic acid salt fraction is prepared. Specifically, the preparation process of the second perfluorobutanesulfonic acid salt fraction includes a process in which hydrogen fluoride gas is removed from the second gaseous component exhausted from the electrochemical fluorination and a process in which perfluorobutanesulfonic acid salt is produced by gas-liquid contacting perfluorobutanesulfonyl fluoride (PBF) in the second gaseous phase component after removal of hydrogen fluoride gas and an alkaline solution.

First, hydrogen fluoride gas is removed from the gaseous phase component exhausted from the electrochemical fluorination cell. Specifically, it is preferable that hydrogen fluoride is removed by washing the gaseous phase component by gas-liquid contacting the component with a shower of water or a low-concentration alkaline solution, since hydrogen fluoride not liquefied by the condenser is included in the exhausted second gaseous phase component.

Next, perfluorobutanesulfonic acid salt is produced by gas-liquid contacting the PBF in the gaseous phase component after the removal of hydrogen fluoride gas to an alkaline solution. Specifically, the gaseous phase component after the removal of hydrogen fluoride gas is introduced to a gas absorbing tower and gas-liquid contacted to an alkaline solution. By following the processes described above, PBF reacts with an alkaline compound in the alkaline solution and is converted to perfluorobutanesulfonic acid salt to be reactively absorbed to the alkaline solution as shown in the formula (4) above.

As the alkaline solution, the alkaline solutions used in the preparation process of the first perfluorobutanesulfonic acid salt fraction can be used.
Also, a surfactant can be mixed to the alkaline solution in order to absorb the produced gas of PBF effectinely to the alkaline solution.

On the other hand, co-produced perfluoroalkanes included in the electrochemically produced gas (gaseous phase component) are not absorbed to the alkaline solution and exhausted to the outside of the system. Also, sulfonyl difluoride (SO₂F₂) is absorbed to the alkaline solution to produce sulfuric acid salt (M₂SO₄) and alkaline metal fluoride (MF).

Next, perfluorobutanesulfonic acid salt is purified by removing impurities after concentrating and drying the alkaline solution absorbed the produced perfluorobutanesulfonic acid salt.
By following the above-described processes, the second perfluorobutanesulfonic acid salt fraction can be prepared.

The perfluorobutanesulfonyl fluoride (C₈F₁₇SO₂F; POF), which is produced by a side reaction of the electrochemical fluorination, is retained in the electrochemical fluorination cell in a state where it is dissolved in both of the reaction solution (hydrofluoric acid) and the product phase (PBF) distributed in an equilibrium. A larger amount of POF is dissolved in PBF compared to hydrofluoric acid.

When the POF content in the reaction solution exceeds 500 ppm, POF is easily exhausted from the electrochemical fluorination cell to the outside of the system as the gaseous phase component accompanied with hydrogen gas produced by electrochemical fluorination. The gaseous phase component is gas-liquid contacted with the alkaline solution as described in the explanation of the preparation process of the second perfluorobutanesulfonic acid salt fraction. Thus, the POF in the gaseous phase component and the alkaline solution react to produce perfluorooctanesulfonic acid salt (PFOS salt). Because of this, when the POF content in the reaction solution exceeds 500 ppm, the produced perfluorobutanesulfonic acid salt fraction is contaminated with a larger amount of the PFOS salt as impurities in the preparation process of the second perfluorobutanesulfonic acid salt fraction.

In the preparation process of the first perfluorobutanesulfonic acid salt fraction describe above, PBF is withdrawn as a liquid phase component and purified further by distillation. Thus, PBF can be easily separated and purified even if it is contaminated with POF at a high concentration.
Contrary to that, POF cannot be removed by distillation, since the product of interest, which is not PBF but perfluorobutanesulfonic acid salt, is recovered in a liquid phase in the preparation process of the second perfluorobutanesulfonic acid salt fraction. Also, separation of POF salt from the perfluorobutanesulfonic acid salt is difficult in a case where the produced perfluorobutanesulfonic acid salt fraction is contaminated with PFOS salt as impurities. Therefore, it is important to avoid the contamination of POF in the gaseous phase component in the preparation process of the second perfluorobutanesulfonic acid salt fraction.

In the process for producing perfluorobutanesulfonic acid salt of the present embodiment, the perfluorooctanesulfonyl (POF) content in the reaction solution in the electrochemical fluorination cell is regulated to be 500 ppm or less by withdrawing the liquid phase component. In other words, the POF dissolved in the liquid phase component abundantly is removed from the electrochemical fluorination cell by withdrawing the liquid phase component produced in the preparing process of the first perfluorobutanesulfonic acid salt fraction. When newly produced PBF is produced as a liquid phase component by electrochemical fluorination, the POF dissolved in the reaction solution (hydrofluoric acid) is transferred to the liquid phase component containing the PBF. As a result, the POF content in the reaction solution is kept at 500 ppm or less. Because of this, the POF content in the gaseous phase component, which is exhausted from the electrochemical fluorination cell accompanied with hydrogen in the preparing process of the second perfluorobutanesulfonic acid salt fraction, can be suppressed to 10 ppm or less.
The POF content in the reaction solution may be set to 100 ppm or more and 500 ppm or less. In a case where the POF content in the reaction solution is kept less than 100 ppm, an action other than the withdrawing the liquid phase component has to be taken, increasing the production cost.

The perfluorobutanesulfonic acid salt-containing composition (the first and second perfluorobutanesulfonic acid salt fractions), which is produced by the process for producing perfluorobutanesulfonic acid salt of the present embodiment, has features described below.

The perfluorobutanesulfonic acid salt concentration in the perfluorobutanesulfonic acid salt-containing composition obtained from the first and second perfluorobutanesulfonic acid salt fractions is 99% or more by mass ratio.

The perfluorooctanesulfonic acid salt (PFOS salt) content of the perfluorobutanesulfonic acid salt-containing composition, which is obtained in the preparing process of the first perfluorobutanesulfonic acid salt fraction, is 5 ppm or less.

On the other the perfluorooctanesulfonic acid salt (PFOS salt) content of the perfluorobutanesulfonic acid salt-containing composition, which is obtained in the preparing process of the second perfluorobutanesulfonic acid salt fraction, is 10 ppm or less.
Also, the perfluorobutanesulfonic acid salt-containing composition, which is obtained in the preparing process of the second perfluorobutanesulfonic acid salt fraction, contains perfluoroalkanesulfonate salts, which is represented by a formula CₙF₂ₙ₊₁SO₃· M (n is an integer of 1 to 3, and M is a cation component) in a concentration of 200 ppm or less. This is due to the perfluoroalkanesulfonyl fluoride, which is co-produced in the electrochemical fluorination process and represented by a formula CₙF₂ₙ₊₁SO₂F (n is an integer of 1 to 3).

As explained above, according to the process for producing perfluorobutanesulfonic acid salt of the present embodiment, among perfluorobutanesulfonyl fluoride (PBF) produced in the electrochemical fluorination, the PBF in the gaseous phase component, which is lost as an exhaust gas conventionally, is collected by gas-liquid contacting the gaseous phase component to the alkaline solution. As a result, the yield of perfluorobutanesulfonic acid salt (C₄F₉SO₃M) obtained by the purification processes afterward can be improved.

Also, perfluorooetanesulfonyl fluoride (POF), which is co-produced by the electrochemical fluorination, is withdrawn as a liquid phase component with the PBF separated to the lower layer side of the reaction solution, and hydrolyzed using an alkaline solution after reducing the POF content in the PBF to 10 ppm by distillation purification. Because of this configuration, the contained amount of perfluorooctanesulfonic acid salt (PFOS salt), which is included in the perfluorobutanesulfonic acid salt-containing composition and a chemical concerned about its environmental accumulation, can be reduced to 10 ppm or less.

Furthermore, the perfluorobutanesulfonic acid salt-containing composition, the PFOS salt content of which is 10 ppm or less, can be produced from the gaseous phase by withdrawing the POF, which is co-produced in the electrochemical fluorinations as a liquid phase component with the PBF separated to the lower layer of the reaction solution at regular intervals and collecting the PBF gas accompanied with hydrogen while the POF content in the reaction solution is kept to 500 ppm or less.

The POF content in the reaction solution can be measured by gas chromatography after treating the reaction solution. The PFOS content and CₙF₂ₙ₊₁SO₃M (n is an integer of 1 to 3) content of the perfluorobutanesulfonic acid salt fractions can be measured by ion chromatography.

### [Examples]

The technical effect of the present invention is explained in more detail using Examples below. However, the present invention is not particularly limited by the description of these Examples.

### [Example 1 and Example 2]

In Examples 1 and 2, the yield of perfluorobutanesulfonyl fluoride (PBF) and the contained amount of the perfluorooetanesulfonic acid salt (PFOS salt) in the produced perfluorobutanesulfonate potassium salt (KFBS salt) are studied by performing the process for producing perfluorobutanesulfonate of the above-described embodiment, to which the present invention is applied.

Specifically, first, PBF was produced by electrochemical fluorination of sulfolane as a material in the process for producing perfluorobutanesulfonic acid salt of the above-described embodiment. Next, the perfluorooctanesulfonyl fluoride (POF) content of the reaction solution was kept at 500 ppm or less by withdrawing the liquid phase component from the electrochemical fluorination cell at regular intervals, and at the same time PBF is recovered as a gaseous phase component. Next, PBF recovered as the liquid and gaseous phase components were hydrolyzed using potassium hydroxide as the alkaline solution, and KFBS salt were produced from the both components. The temperature of the coolant in the condenser was -20°C in the electrochemical fluorination process.
The yield of KFBS salt from the material, and the contained amounts of PFOS salt in the reaction solution and in the produced KFBS salt are shown in TABLE 1.

### [Comparative Example 1]

Comparative Example 1 differs from Example 1 of the present invention in not recovering the gaseous phase component of the PBF produced by the reaction solution fluorination. The result of Comparative Example 1 is also shown in TABLE 1.

### [Comparative Examples 2 and 3]

Comparative Examples 2 and 3 differ from Example 1 of the present invention in not keeping the POF content in the reaction solution to 500 ppm or less. The results of Comparative Examples 2 and 3 are also shown in TABLE 1.

**[TABLE 1]**

| | POF content in the reaction solution (ppm) | Contained amount of PFOS salt in salt from the liquid phase PBF (ppm) | Contained amount of PFOS salt KFBS in KFBS salt from the gaseous phase (ppm) PBF | Contained amount of C1-C3 salt compound in KFBS originated from the gaseous phase (ppm) | Yield from the starting material (%) | | |
|---|---|---|---|---|---|---|---|
| | | | | | KFBS salt originated from the liquid PBF | KFBS salt originated from the gaseous PBF | Total |
| Example 1 of the present invention | 100-30 | 8 | 5 | 150 | 25 | 19 | 44 |
| Example 2 of the present invention | 300-500 | 9 | 5 | 160 | 24 | 18 | 42 |
| Comparative Example 1 | 100-300 | 8 | - | - | 25 | 0 | 25 |
| Comparative Example 2 | 500-700 | 9 | 55 | 170 | 26 | 17 | 43 |
| Comparative Example 3 | 1700-1900 | 8 | 60 | 150 | 24 | 19 | 43 |

As shown in TABLE 1, in Example 1 of the present invention, the yield or KFBS salt in the liquid phase PBF was 25%, and the total yield of KFBS salt was 44% after combining it to the KFBS salt originated from the gaseous phase PBF, since PBF, which is produced by the electrochemical fluorination, is recovered as the liquid phase component and the gaseous phase component. Similarly, the total yield of KFBS salt in Example 2 of the resent invention was 42%.

Furthermore, both of the contained amounts of PFOS salt in KFBS salt originated from the liquid phase PBF and the gaseous phase PBF were suppressed to 10 ppm or less in Examples 1 and 2 of the present invention, by withdrawing the liquid phase component at regular intervals to recover PBF, and keeping the POF content in the reaction solution at 100 to 300 ppm in Example 1 or at 300 to 500 ppm in Example 2.

Contrary to these results, the total yield was 25%, which corresponds to the KFBS salt originated from the liquid PBF alone, in Comparative Example 1.

Also, the contained amount of PFOS salt in KFBS salt originated from the gaseous phase PBF was 55 ppm in Comparative Example 2, since the POF content in the reaction solution was 500 to 700 ppm and higher than 500 ppm.

Also, the contained amount of the PFOS salt in KFBS salt originated from the gaseous PBF was 60 ppm in Comparative Example 3, since the POF content in the reaction solution was 1700 to 1900 ppm and higher than 500 ppm.

### INDUSTRIAL APPLICABILITY

The yield of perfluorobutanesulfonic acid salt can be improved since PBF, which has been exhausted from the electrochemical fluorination cell conventionally as a gaseous phase component to the outside of the system, is collected and converted to perfluorobutanesulfonic acid salt. Also, the contained amount of POF included in the gaseous phase component can be reduced, since the POF content in the reaction solution in the electrochemical fluorination cell is controlled to be 500 ppm or less. Because of this, the contained amount of PFOS salt in the perfluorobutanesulfonic acid salt fraction produced from the gaseous phase component can be suppressed to 10 ppm or less.

## Claims

1. A process for producing perfluorobutanesulfonic acid salt comprising the steps of:
electrochemical fluorination, in which a liquid phase component and a gaseous phase component containing perfluorobutanesulfonyl fluoride are generated by electrochemical fluorination a starting material consisting of one or more selected from the group consisting of butane sulfonyl fluoride, tetrahydrohiophene- 1,1 -dioxide (sulfolane), and 2,5-dihydrohiophene-1,1-dioxide (sulfolene) in a reaction solution made of anhydrous hydrogen fluoride in electrochemical fluorination cell;
preparing a first perfluorobutanesulfonate fraction, in which the first perfluorobutanesulfonate fraction is prepared by withdrawing the liquid phase component generated in the electrochemical fluorination cell in the step of electrochemical fluorination and hydrolyzing the perfluorobutanesulfonyl fluoride contained in the liquid phase component; and
preparing a second perfluorobutanesulfonate fraction, in which the second perfluorobutanesulfonate fraction is prepared by collecting the gaseous phase component that is exhausted from the electrochemical fluorination cell and generated in the step of electrochemical fluorination and hydrolyzing the perfluorobutanesulfonyl fluoride contained in the gaseous phase component, wherein
a perfluorobutanesulfonyl fluoride content in the reaction solution is controlled to be 500 ppm or less by withdrawing the liquid phase component from the electrochemical fluorination cell in the step of electrochemical fluorination.

2. The process for producing perfluorobutanesulfonic acid salt according to Claim 1, wherein:
the step of preparing the first perfluorobutanesulfonate fraction comprises a process of purifying perfluorobutanesulfonyl fluoride, in which perfluorobutanesulfonyl fluoride is purified by distilling the liquid phase component before hydrolyzing the perfluorobutanesulfonyl fluoride contained in the liquid phase component; and
the purified perfluorobutanesulfonyl fluoride is hydrolyzed by an alkaline solution.

3. The process for producing perfluorobutanesulfonic acid salt according to Claim 1 or 2, wherein:
the step of preparing the second perfluorobutanesulfonate fraction comprises a process of removing hydrogen fluoride gas, in which hydrogen fluoride gas is removed from the gaseous phase component before hydrolyzing the perfluorobutanesulfonyl fluoride contained in the gaseous phase component;
the perfluorobutanesulfonyl fluoride in the gaseous phase component, the hydrogen fluoride gas of which being removed, is hydrolyzed by gas-liquid contacting to the alkaline solution; and
the second perfluorobutanesulfonate fraction is collected by the alkaline solution.

4. The process for producing perfluorobutanesulfonic acid salt according to any one of Claims 1 to 3, wherein a perfluorooctanesulfonic acid salt (PFOS salt) content in the first perfluorobutanesulfonate fraction and a perfluorooctanesulfonyl fluoride salt (PFOS salt) content in the second perfluorobutanesulfonate fraction are 50 ppm or less.

5. The process for producing perfluorobutanesulfonic acid salt according to any one of Claims 1 to 3, wherein a perfluorooctanesulfonic acid salt (PFOS salt) content in the first perfluorobutanesulfonate fraction and a perfluorooctanesulfonic acid salt (PFOS salt) content in the second perfluorobutanesulfonate fraction are 10 ppm or less.

6. A perfluorobutanesulfonic acid salt-containing composition produced by the method according to any one of Claims 1 to 3, wherein:
a perfluorobutanesulfonic acid salt concentration in the perfluorobutanesulfonic acid salt-containing composition is 99% by mass or more; and
a perfluorooctanesulfonic acid salt (PFOS salt) content is 50 ppm or less.

7. The perfluorobutanesulfonic acid salt-containing composition produced by the method according to any one of Claims 1 to 3, wherein a perfluorooctanesulfonic acid salt (PFOS salt) content is 10 ppm or less.

8. The perfluorobutanesulfonic acid salt-containing composition produced by the method according to any one of Claims 1 to 3 containing 200 ppm or less of a perfluoroalkanesulfonate salt represented by a formula CₙF₂ₙ₊₁SO₃. M, wherein n indicates an integer from 1 to 3, and M indicates a cation component.
